# EUROPEAN PATENT SPECIFICATION

(11) **EP 4 156 195 B1**
(45) Date of publication and mention of the grant of the patent: **30.10.2024**
(21) Application number: 22197012.2
(22) Date of filing: 22.09.2022
(51) Int. Cl.: G16H 40/67, G16H 20/17, G16H 40/40, A61M 5/14, A61M 5/168, A61M 5/172, A61M 5/315, G16H 40/63

(54) **MACHINE LEARNING ENABLED DETECTION OF INFUSION PUMP MISLOADS**
DURCH MASCHINENLERNEN ERMÖGLICHTE ERKENNUNG VON INFUSIONSPUMPENFEHLLASTEN
DÉTECTION DE CHARGES ERRONÉES DE POMPE À PERFUSION ACTIVÉE PAR APPRENTISSAGE AUTOMATIQUE

(30) Priority: 24.09.2021 US 202163248069 P
(43) Date of publication of application: 29.03.2023
(73) Proprietor: CareFusion 303, Inc., San Diego, CA 92130 (US)
(72) Inventor: ABAL, Daniel, San Diego, 92130 (US); BURGESS, Brendan John, San Diego, 92130 (US); SUBRAMANIAN, Ramkumar, San Diego, 92130 (US)
(74) Representative: Schulz, Oliver Frank Michael

(56) References cited:
- CN-A- 110 838 118
- JP-A- 2020 531 940
- US-A1- 2014 318 639
- US-A1- 2021 059 784
- ZAN GAO ET AL: "Multi-camera Monitoring of Infusion Pump Use", SEMANTIC COMPUTING (ICSC), 2010 IEEE FOURTH INTERNATIONAL CONFERENCE ON, IEEE, PISCATAWAY, NJ, USA, 22 September 2010 (2010-09-22), pages 105 - 111, XP031795795, ISBN: 978-1-4244-7912-2
- HWANG YOUNG JUN ET AL: "Convolutional neural network-based ambient light-independent panel digit surveillance technique for infusion pumps", PROCEEDINGS OF THE INSTITUTION OF MECHANICAL ENGINEERS.JOURNAL OF ENGINEERING IN MEDICINE. PART H., vol. 235, no. 5, 21 May 2021 (2021-05-21), GB, pages 566 - 573, XP093017290, ISSN: 0954-4119, DOI: 10.1177/0954411921996090

## Description

### TECHNICAL FIELD

The subject matter described herein relates generally to machine learning and more specifically to machine learning based techniques for detecting misloading of an infusion pump.

### BACKGROUND

A fluid pump, such as an infusion pump, may be used to administer therapy to a patient by delivering nutrients, medications, blood products, or other substance to the patient. Many types of clinical treatments, such as pain management, blood glucose regulation, and chemotherapy, may require the delivery of precise volumes of fluids to a patient's circulatory system or epidural space via, for example, intravenous infusion, subcutaneous infusion, arterial infusion, epidural infusion, and/or the like. A peristaltic pump (or roller pump) is one example of a fluid pump capable of delivering precise volumes of fluids. For example, a peristaltic pump may be configured to deliver, continuously or intermittently, precisely measured doses of a fluid from a reservoir. The pumping mechanism in the peristaltic pump may include a combination of pumping fingers and occluding fingers that operate in tandem to apply pressure to sequential locations in a tubing (or other conduit) in fluid communication with the reservoir in order to drive the fluid from the reservoir to a patient.

From CN 110 838 118 A systems and methods are known for anomaly detection of a medical procedure based on obtained images and a trained machine learning model.

### SUMMARY

Systems and infusion pumps are provided for detecting an improperly loaded infusion set (e.g., misload) on at an infusion pump. The scope of the invention is defined by the independent claims. Preferred embodiments are depicted in the dependent claims, the description and the accompanying Figures. For example, a pump controller may apply a machine learning model trained to detect, based on one or more images of an infusion pump loaded with an intravenous (IV) set, a misload of the intravenous set. The images of the pump loaded with the intravenous set may be captured by one or more cameras having a field of view (FOV) that includes the portion of the pump with the loaded intravenous set. The machine learning model may be trained using images of correctly loaded intravenous sets and is therefore able to determine when the images of the pump loaded with the intravenous set exhibit one or more nonconformities. When the output of the machine learning model indicates a misload of the intravenous set at the infusion pump, the pump controller may perform one or more corrective actions. For instance, the pump controller may prevent the infusion pump from performing an infusion when a misload of the intravenous set is detected at the pump. Furthermore, the pump controller may generate a message indicating a misload of the intravenous set at the infusion pump. In some cases, the message may identify the type and location of the misload at the infusion pump.

In one aspect, there is provided a system that includes at least one processor and at least one memory. The at least one memory includes program code that provides operations when executed by the at least one processor. The operations include: receiving, from a camera at an infusion pump, one or more images of an area of interest of the infusion pump loaded with an infusion set; identifying, within the one or more images, a first component of the infusion pump and a second component of the infusion set in the area of interest, wherein the first component comprises a bezel, a membrane seal, a door, a platen, a locator feature, or an air-in-line detector, and wherein the second component comprises an upper fitment, a lower fitment, or a pumping segment of a tubing; applying a machine learning model trained to detect one or more nonconformities present in the one or more images of the infusion pump loaded with the infusion set based at least on a relative position of the first component and the second component and by at least comparing, to one or more images of a correctly loaded infusion set, the one or more images of the infusion pump loaded with the infusion set; and in response to an output of the machine learning model indicating a presence of a nonconformity in the one or more images of the infusion pump loaded with the infusion set, performing a corrective action.

In some variations, one or more features disclosed herein including the following features can optionally be included in any feasible combination.

In some variations, the first component and the second component may be identified by applying an edge detection technique.

In some variations, the edge detection technique may include a Laplace-Gaussian transformation and/or a Canny edge detection.

In some variations, the first component and the second component may be identified based on a graphical feature disposed on each one of the first component and the second component.

In some variations, the graphical feature may include a pattern, a barcode, and/or an April tag.

In some variations, the one or more nonconformities may include a misload of the infusion set. The misload of the infusion set may include a misplacement of an upper fitment of the infusion set, a lower fitment of the infusion set, and/or a pumping segment of a tubing of the infusion set.

In some variations, the one or more nonconformities may include the intravenous set being reused, past an expiration date, an incorrect type, or a counterfeit product.

In some variations, the one or more nonconformities may include one or more components of the infusion pump and/or the intravenous set being missing.

In some variations, the one or more nonconformities may include one or more components of the infusion pump and/or the intravenous set being damaged.

In some variations, the one or more nonconformities may include a presence of contaminants in the infusion pump and/or the intravenous set.

In some variations, the camera may be mounted to a door of the infusion pump. The camera may be mounted in a location where the camera has a field of view that includes at least a portion of the infusion pump loaded with the intravenous set.

In some variations, the field of view of the camera may exclude one or more areas surveillance is unsuitable, prohibited, or unnecessary.

In some variations, the one or more images may include a first image captured while the door is in an open position and a second image captured while the door is in a partially open position. The one or more nonconformities may be detected based on the first image and the second image.

In some variations, the camera may include a visible light camera, an infrared camera, and/or an ultraviolet camera.

In some variations, the one or more nonconformities may be detected based on a graphical feature that is detectable under visible light, infrared light, or ultraviolet light.

In some variations, the corrective action may include preventing the infusion pump from performing an infusion.

In some variations, the corrective action may include generating a message identifying the one or more nonconformities.

In another aspect, there is provided an infusion pump including a bezel, a camera, and a controller. The bezel includes one or more locator features for receiving an intravenous set. The camera is mounted to a door of the infusion pump. The camera is mounted in a location where the camera has a field of view that includes at least a portion of the infusion pump loaded with the intravenous set. The camera is configured to capture one or more images of the infusion pump loaded with the intravenous set. The controller includes at least data processor and at least one memory storing instructions which, when executed by the at least one data processor, cause the controller to perform operations. The operations include: identifying, within the one or more images, a first component of the infusion pump and a second component of the infusion set within the area of interest, the first component comprising the bezel, the door, the one or more locator features, a membrane seal, a platen, or an air-in-line detector, and wherein the second component comprises an upper fitment, a lower fitment, or a pumping segment of a tubing; applying a machine learning model trained to detect one or more nonconformities present in the one or more images of the infusion pump loaded with the infusion set based at least on a relative position of the first component and the second component and by at least comparing, to one or more images of a correctly loaded infusion set, the one or more images of the infusion pump loaded with the infusion set; and in response to an output of the machine learning model indicating a presence of a nonconformity in the one or more images of the infusion pump loaded with the infusion set, performing a corrective action.

In some variations, one or more features disclosed herein including the following features can optionally be included in any feasible combination.

In some variations, the first component and the second component are identified by applying an edge detection technique.

In some variations, the first component and the second component may be identified by applying an edge detection technique.

In some variations, the edge detection technique may include a Laplace-Gaussian transformation and/or a Canny edge detection.

In some variations, the first component and the second component may be identified based on a graphical feature disposed on each one of the first component and the second component.

In some variations, the graphical feature may include a pattern, a barcode, and/or an April tag.

In some variations, the one or more nonconformities may include a misload of the infusion set. The misload of the infusion set may include a misplacement of an upper fitment of the infusion set, a lower fitment of the infusion set, and/or a pumping segment of a tubing of the infusion set.

In some variations, the one or more nonconformities may include the intravenous set being reused, past an expiration date, an incorrect type, or a counterfeit product.

In some variations, the one or more nonconformities may include one or more components of the infusion pump and/or the intravenous set being missing.

In some variations, the one or more nonconformities may include one or more components of the infusion pump and/or the intravenous set being damaged.

In some variations, the one or more nonconformities may include a presence of contaminants in the infusion pump and/or the intravenous set.

In some variations, the field of view of the camera may further exclude one or more areas surveillance is unsuitable, prohibited, or unnecessary.

In some variations, the one or more images may include a first image captured while the door is in an open position and a second image captured while the door is in a partially open position. The one or more nonconformities may be detected based on the first image and the second image.

In some variations, the camera may include a visible light camera, an infrared camera, and/or an ultraviolet camera.

In some variations, the one or more nonconformities may be detected based on a graphical feature that is detectable under visible light, infrared light, or ultraviolet light.

In some variations, the corrective action may include preventing the infusion pump from performing an infusion.

In some variations, the corrective action may include generating a message identifying the one or more nonconformities.

By means of example, not part of the claimed invention, methods consistent with the descriptions provided herein as well as articles that comprise a tangibly embodied machine-readable medium operable to cause one or more machines (e.g., computers, etc.) to result in operations implementing one or more of the described features are described. Similarly, computer systems are also described that may include one or more processors and one or more memories coupled to the one or more processors. A memory, which can include a non-transitory computer-readable or machine-readable storage medium, may include, encode, store, or the like one or more programs that cause one or more processors to perform one or more of the operations described herein. Computer implemented methods consistent with one or more implementations of the current subject matter can be implemented by one or more data processors residing in a single computing system or multiple computing systems. Such multiple computing systems can be connected and can exchange data and/or commands or other instructions or the like via one or more connections, including, for example, to a connection over a network (e.g. the Internet, a wireless wide area network, a local area network, a wide area network, a personal area network, a peer-to-peer network, a personal area network, a peer-to-peer network, a mesh network, a wired network, or the like), via a direct connection between one or more of the multiple computing systems, etc.

The details of one or more variations of the subject matter described herein are set forth in the accompanying drawings and the description below. Other features and advantages of the subject matter described herein will be apparent from the description and drawings, and from the claims. While certain features of the currently disclosed subject matter are described for illustrative purposes in relation to detecting a tubing misload at an infusion pump, it should be readily understood that such features are not intended to be limiting. The claims that follow this disclosure are intended to define the scope of the protected subject matter.

### DESCRIPTION OF DRAWINGS

The accompanying drawings, which are incorporated in and constitute a part of this specification, show certain aspects of the subject matter disclosed herein and, together with the description, help explain some of the principles associated with the disclosed implementations. In the drawings,
FIG. 1A depicts a system diagram illustrating an example of an infusion system, in accordance with some example embodiments;
FIG. 1B depicts a block diagram illustrating an example of a workflow associated with an infusion system, in accordance with some example embodiments;
FIG. 2A depicts a perspective view of an example of an infusion pump, in accordance with some example embodiments;
FIG. 2B depicts another perspective view of an example of an infusion pump, in accordance with some example embodiments;
FIG. 2C depicts a side view of an example of an infusion pump, in accordance with some example embodiments;
FIG. 3A depicts a front view of an infusion pump with an example of a misloaded intravenous set, in accordance with some example embodiments;
FIG. 3B depicts another front view of an infusion pump with an example of a misloaded intravenous set, in accordance with some example embodiments;
FIG. 4 depicts a front view of an infusion pump with another example of a misloaded intravenous set, in accordance with some example embodiments;
FIG. 5A depicts an example of a raw image used for machine learning enabled misload detection, in accordance with some example embodiments;
FIG. 5B depicts an example of an enhanced image used for machine learning enabled misload detection, in accordance with some example embodiments;
FIG. 6A depicts an example of a raw image used for machine learning enabled misload detection, in accordance with some example embodiments;
FIG. 6B depicts an example of an enhanced image used for machine learning enabled misload detection, in accordance with some example embodiments;
FIG. 7A depicts an example of a raw image used for machine learning enabled misload detection, in accordance with some example embodiments;
FIG. 7B depicts an example of an enhanced image used for machine learning enabled misload detection, in accordance with some example embodiments;
FIG. 8A depicts an example of a raw image used for machine learning enabled misload detection, in accordance with some example embodiments;
FIG. 8B depicts an example of an enhanced image used for machine learning enabled misload detection, in accordance with some example embodiments;
FIG. 9A depicts a close-up view of a portion of an infusion pump with some examples of graphical features, in accordance with some example embodiments;
FIG. 9B depicts a front view of an infusion pump with some additional examples of graphical features, in accordance with some example embodiments;
FIG. 10 depicts a flowchart illustrating an example of a process for machine learning enabled misload detection, in accordance with some example embodiments; and
FIG. 11 depicts a block diagram illustrating an example of a computing system, in accordance with some example embodiments.

When practical, similar reference numbers denote similar structures, features, or elements.

### DETAILED DESCRIPTION

An infusion pump, such as a peristaltic pump, may fail to operate correctly or as expected (e.g., according to the delivery parameters configuring the device such as rate or volume to be infused) if an intravenous (IV) set is not correctly loaded in the pump. Misloads can occur when the tubing of the intravenous set is placed in an incorrect position relative to various components of the pump including, for example, the pump's pumping mechanism. For example, the proper operation of the pump may require the tubing to be stretched and centered relative to a pumping finger. A misload in which the tubing is not stretched a sufficient amount may prevent the pumping fingers from closing properly against the tubing and cause an uncontrolled flow of fluid from the pump. Meanwhile, a misload that causes an excessive stretching of the tubing may also cause an uncontrolled flow of fluid from the pump. For instance, overstretching the tubing may reduce the thickness of the walls of the tubing and prevent the occluding fingers from closing off the tubing to stop the flow of fluid.

In some cases, a misload of the intravenous set may also introduce inaccuracies in the flow rate of the pump by changing the cross-sectional dimensions of the tubing. For example, the tubing may be twisted along the longitudinal axis. Alternatively, the inner diameter of the tubing may narrow when the upper fitment of the intravenous set is not placed on the base of the pocket in the pump bezel but is placed above the pocket in the pump bezel. In the event the infusion pump includes a platen, either as a part of the door of the pump or as a separate component, a misload may occur if a foreign object, such as a misplaced portion of the intravenous set, is present in an area reserved for the pumping segment of the tubing. In addition to discrepancies in the flow rate of the pump, the presence of the foreign object may cause breakages in the intravenous set and the pump, such as the platen, when excessive force is used to closed the door of the pump.

Any of the aforementioned types of anomalous conditions may cause an infusion pump to deliver incorrect quantities of fluids or malfunction. As such, it may be desirable to detect a misloaded intravenous set at the infusion pump before the infusion commences. However, conventional approaches to detecting a misloaded intravenous set that rely on electromechanical switches pose a number of limitations. For example, electromechanical switches are able to detect nonconformities at a limited quantity of locations within the infusion pump while the flexibility of the tubing in the intravenous set lends multiple degrees of freedom to the placement of the intravenous set. As such, conventional misload detection approaches that rely on electromechanical switches may be neither reliable nor practical. For instance, electromechanical switches may be prone to false negatives as well as false positives. Moreover, the inclusion of electromechanical switches in the infusion pump may introduce complexities in the housing and wiring of the infusion pump. The cavities needed to accommodate the electromechanical switches, for example, may be susceptible to the ingress of contaminants but difficult to clean.

In some example embodiments, a pump controller may be configured to detect a misloaded intravenous set based on one or more images of a pump loaded with an intravenous set. For example, the pump controller may apply a machine learning model that has been trained based on images of correctly loaded intravenous sets to determine when the images of the pump loaded with the intravenous set exhibit one or more nonconformities. When the output of the machine learning model indicates a misload of the intravenous set at the infusion pump, the pump controller may perform one or more corrective actions. For instance, the pump controller may prevent the infusion pump from performing an infusion when a misload of the intravenous set is detected at the pump. Alternatively and/or additionally, the pump controller may generate a message indicating a misload of the intravenous set at the infusion pump. In some cases, the message may provide instructions for correcting the misload including by identifying the type and location of the misload at the infusion pump.

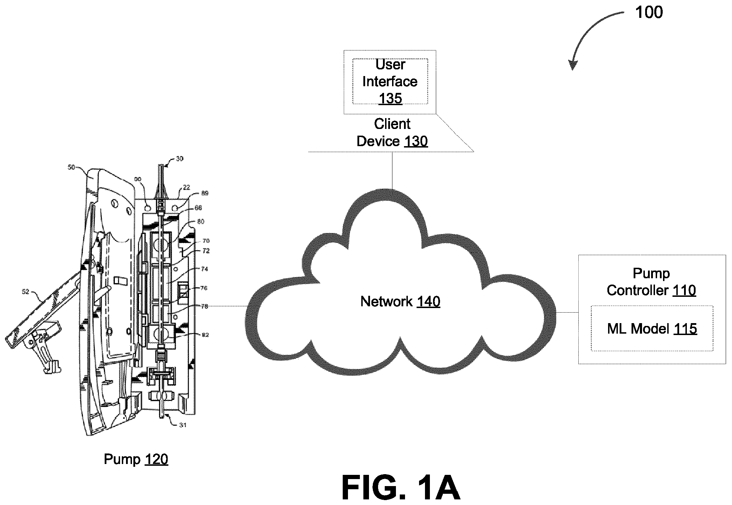

FIG. 1A depicts a system diagram illustrating an example of an infusion system 100, in accordance with some example embodiments. Referring to FIG. 1A, the infusion system 100 may include a pump controller 110, a pump 120, and a client device 130. As shown in FIG. 1A, the pump controller 110, the pump 120 and the client device 130 may be communicatively coupled via a network 140. The client device 130 may be a processor-based device including, for example, a point of care unit (PCU), a smartphone, a tablet computer, a wearable apparatus, a desktop computer, a laptop computer, a workstation, and/or the like. Meanwhile, the network 140 may be a wired and/or wireless network including, for example, a public land mobile network (PLMN), a local area network (LAN), a virtual local area network (VLAN), a wide area network (WAN), the Internet, and/or the like.

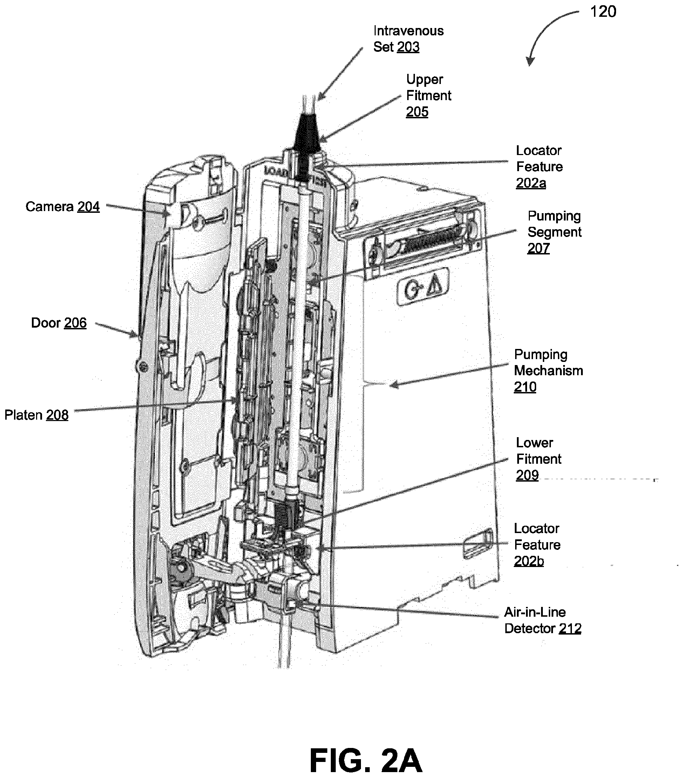

FIGS. 2A-C depict various views of an example of the pump 120 with a door 206 open. As shown in FIGS. 2A-B, the pump 120 may be loaded with an intravenous (IV) set 203. The pump 120 may act directly on a pump segment 207 of the intravenous set 203, which connects an upstream fluid line to a downstream fluid line to form a continuous fluid conduit between a fluid reservoir and a patient. For example, the pump 120 may include a pumping mechanism 210, which may act as a flow control device moving fluid through the conduit downstream to the patient. The pump segment 207, the upstream fluid line, and/or the downstream fluid line may be coupled to a pump cassette or cartridge that is configured to be coupled to the pump 120.

Although the type of the pumping mechanism 210 may vary, the example of the pumping mechanism 210 shown in FIGS. 2A-C is a multi-finger pumping mechanism that includes an upstream occluding finger, a primary pumping finger, a downstream occluding finger, and a secondary pumping finger. The pumping mechanism 210 may therefore operate by the pumping fingers and the occluding fingers alternately applying pressure on the pump segment 207 of the fluid conduit. The pressure may be applied at sequential locations in the pump segment 207, beginning at the upstream end of the pumping mechanism 210 and continuing through the downstream end of the pumping mechanism 210. At any one point in time, at least one of the fingers of the pumping mechanism 210 may apply a pressure that is sufficient to occlude the fluid conduit. Moreover, at least one of the fingers of the pumping mechanism 210 may not retract from occluding the pump segment 207 until a subsequent finger in the sequence has already occluded the pump segment 207. Accordingly, at no time during the operation of the pump mechanism 210 is there a direct fluid path from the reservoir to the patient.

In some example embodiments, the pump controller 110 may be configured to detect a misload of the intravenous set 203 based at least on one or more images of the pump 120 loaded with the intravenous set 203. For example, the pump controller 110 may include a machine learning engine 115 configured to determine whether the images of the pump 120 loaded with the intravenous set 203 exhibit one or more nonconformities. Moreover, when the output of the machine learning engine 115 indicates a misload of the intravenous set 203, the pump controller 110 may perform one or more corrective actions. For instance, the pump controller 110 may prevent the pump 120 from performing an infusion when a misload of the intravenous set 203 is detected at the pump 120. Alternatively and/or additionally, the pump controller 110 may generate a message indicating a misload of the intravenous set 203 at the pump 120. In some cases, the message may provide instructions for correcting the misload including by identifying the type and location of the misload at the pump 120.

The machine learning engine 115 may be include one or more machine learning models that have been trained based on images of correctly loaded intravenous sets. For example, the machine learning engine 115 may be implemented using one or more general purpose central processing units (CPUs) and/or machine learning hardware accelerators. The one or more machine learning models may be trained to perform a variety of machine vision tasks including, for example, image enhancement, morphological filtering, blob detection, feature extraction, image segmentation, edge detection, object classification, optical character recognition (OCR), and/or the like. For instance, the one or more machine learning models may be trained based on a training set of reference images depicting correctly loaded intravenous set and/or incorrectly loaded intravenous sets. Moreover, the one or more machine learning models may be update based on data collected from real world clinical settings including, for example, images of intravenous set that clinicians identify as being correctly loaded. Examples of such machine learning models include a neural network, a regression model, an instance-based model, a regularization model, a decision tree, a random forest, a Bayesian model, a clustering model, an associative model, a dimensionality reduction model, and/or an ensemble model.

In some example embodiments, the pump 120 may include a camera 204 configured to generate one or more images of the pump 120 loaded with the intravenous set 203. It should be appreciated that the camera 204 may include a variety of image sensors including, for example, a charge-coupled device (CCD), an active-pixel sensor (or complementary metal-oxide-semiconductor (CMOS) sensor), and/or the like. Moreover, the camera 204 may be configured to detect wavelengths of light that occupy different portions of the electromagnetic spectrum including, for example, visible light (400-700 nanometers), infrared light (700-15,000 nanometers), ultraviolet light (10-400 nanometers), and/or the like.

The camera 204 may be configured to have a minimum field of view (FOV) such as, for example, 130° or the like. Moreover, the camera 204 may be mounted to in a location such that the field of view (FOV) of the camera 204 includes one or more areas of interest. In the example shown in FIGS. 2A-C, the camera 204 may be mounted to the door 206, for example, near the edge of the door 206, where the field of view of the camera 204 includes at least a portion of the platen 208 and the intravenous set 203 loaded in the pump 120 (e.g., the portion of the intravenous set 203 between an upper fitment 205 and a lower fitment 209) when the door 206 of the pump 120 is in an open position. According to some example embodiments, the field of view of the camera 204 may be limited to exclude certain areas including, for example, areas where surveillance is unsuitable, prohibited, or unnecessary.

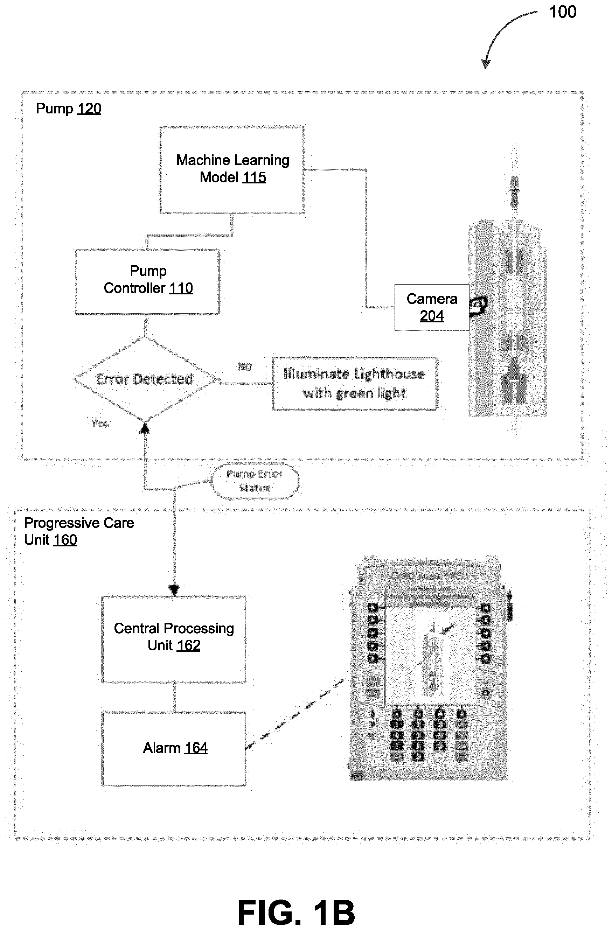

To further illustrate, FIG. 1B depicts a block diagram illustrating an example of a workflow associated with the infusion system 110, in accordance with some example embodiments. As shown in FIG. 1B, the machine learning engine 115 may receive, from the camera 204, one or more images of the pump 120 loaded with the intravenous set 203. The camera 204 may be configured to capture images, for example, periodically, while the door 206 of the pump 120 is in an open position (or partially open position). The machine learning engine 115 generate an output indicating whether the one or more images of the pump 120 loaded with the intravenous set 203 exhibits one or more nonconformities including, for example, a misload of the intravenous set 203.

As shown in FIG. 1B, the pump controller 110 may perform, based at least on the output of the machine learning engine 115, one or more corresponding actions. For example, in the event the output of the machine learning engine 115 indicates that the images of the pump 120 loaded with the intravenous set 203 do not exhibit any nonconformities, the pump controller 110 may allow the pump 120 to perform an infusion and generate a corresponding output (e.g., illuminate a first colored light at the pump 120). Alternatively, if the output of the machine learning engine 115 indicates the presence of a nonconformity in the images of the pump 120 loaded with the intravenous set 203, the pump controller 110 may prevent the pump 120 from performing an infusion.

In the example of the workflow shown in FIG. 1B, the pump controller 110 may interact with a central processing unit 162 at a point of care unit (PCU) 160 in order to trigger an alarm 164. The alarm 164 may include a message indicating a misload of the intravenous set 203 at the pump 120. Furthermore, in some cases, the message may provide instructions for correcting the misload including by identifying the type and location of the misload at the pump 120. For instance, the message may include an image within one or more indicators of the type and location of the misload 120. In the event the machine learning engine 115 is unable to generate an output based on the images captured by the camera 204, for example, due to various errors at the camera 204 (e.g., the door 206 not being in a sufficiently open position, poor lighting conditions, obstruction by a foreign object, and/or the like), the pump controller 110 may also generate a message with instructions to correct the error.

In some example embodiments, the pump controller 110 may track the occurrence of nonconformities, such as misloaded intravenous sets, for individual clinicians and groups of clinicians. If a threshold quantity of nonconformities are detected while a clinician is loading the pump 120, for example, the pump controller 110 may generate a message for another clinician to provide assistance with the loading of the pump 120. Alternatively and/or additionally, in the event nonconformities occur at an above threshold frequency, the pump controller 110 may generate a recommendation for additional training, for example, for an individual clinician or a group of clinicians.

In some example embodiments, the machine learning engine 115 may be configured to detect nonconformities that are present in multiple images captured by the camera 204. For example, the camera 204 may capture a series of images while the door 206 of the pump 120 is being moved from an open position to a closed position including, for example, a first image of the pump 120 loaded with the intravenous set 203 while the door 206 is in an open position and a second image of the pump 120 loaded with the intravenous set 203 while the door 206 is in a partially open (or closed) position. Moreover, the machine learning engine 115 may determine, based at least on the first image and the second image, whether a nonconformity is present in the loading of the intravenous set 203. For instance, the first image taken while the door 206 is in the open position may permit an analysis of the placement of the upper fitment 205 and the lower fitment 209 but how the platen 208 engages with the pumping mechanism 210 and a pumping segment 207 of a tubing of the intravenous set 203 may not be discernable from the first image. As such, the machine learning engine 115 may analyze the first image and the second image in order to detect nonconformities associated with the upper fitment 205, the lower fitment 209, and the platen 208.

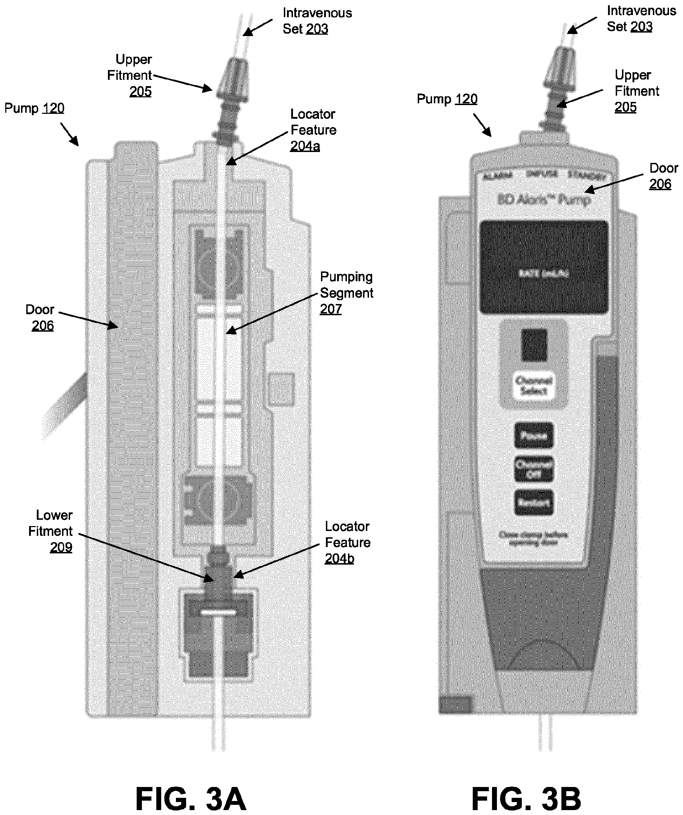

The machine learning engine 115 may be configured to detect a variety of nonconformities present in the images of the pump 120 loaded with the intravenous set 203. A misload of the intravenous set 203 in which one or more portions of the intravenous set 203 are placed incorrectly in the pump 120, such as the misplacement of the upper fitment 205, the pumping segment 207, and/or the lower fitment 209, is one example of a nonconformity. FIGS. 3A-B depict one example of a misload in which the upper fitment 205 of the intravenous set 203 is not in a correct position at the base of a first locator feature 202a (e.g., a pocket) in the bezel of the pump 120 but is placed above the first locator feature 202a. When the upper fitment 205 of the intravenous set 203 is displaced in this manner, the tubing of the intravenous set 203, for example, the pumping segment 207, may stretch to a narrower inner diameter. This change in the cross-sectional dimensions of the tubing may introduce inaccuracies in the flow rate of the pump 120 and cause errors such as under infusion or over infusion.

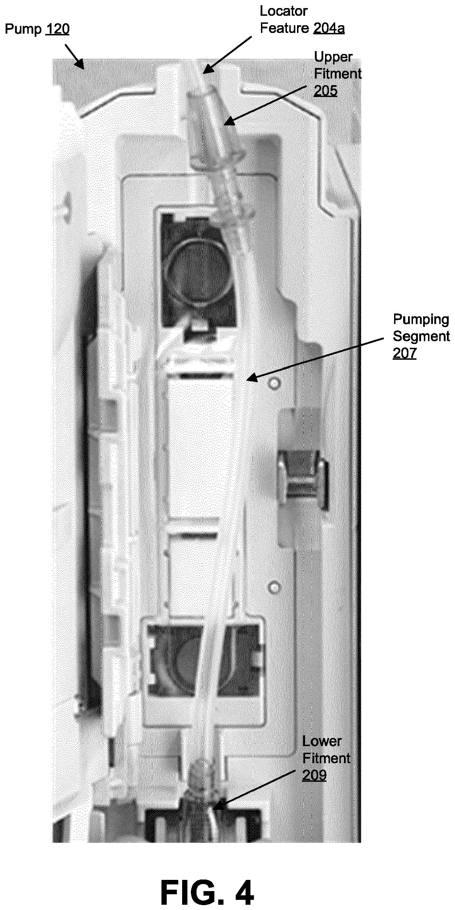

FIG. 4 depicts another example of a misload in which the upper fitment 205 of the intravenous set is placed below the first locator feature 202a. As shown in FIG. 4, this type of misload may cause the pumping segment 207 of the intravenous set 203 to deform and not engage properly with the pumping mechanism 210 of the pump 120. The warped portion of the pumping segment 207 may also prevent the platen 208 from engaging with the pumping mechanism 210 of the pump 210 when the door 206 is in the closed position. Breakages may occur in the intravenous set 203 and the pump 120 if excessive force is used to close the door 206 against the warped section of the pumping segment 207.

Other examples of misloads may include the lower fitment 209 of the intravenous set 203 not being placed in a correct position relative to a second locator feature 202b, an open flow stop, the tubing of the intravenous set 203 not engaged with an air-in-line detector 212 of the pump 120, and the presence of foreign objects in the pumping mechanism 210. In some cases, the misload may be the intravenous set 203 being of an incorrect type, a counterfeit product, or an expired set. For example, the machine learning engine 115 may be trained to identify and differentiate between a standard set and an epidural set, in which case the machine learning engine 115 may detect a nonconformity when the intravenous set 203 is a standard set but the clinical procedure being performed requires an epidural set instead. The type of the intravenous set 203, the expiration date for the intravenous set 203, and whether the intravenous set 203 is a genuine product may be detected based on graphical features on the intravenous set 203 (e.g., patterns, barcodes, and/or the like), which may be detectable under visible light or under special lighting conditions (e.g., ultraviolet light). For instance, the barcode (or other unique identifier) associated with the intravenous set 203 may be used to query a database and determine whether the intravenous set 203 is a genuine product. In some cases, the database may track the usage of the intravenous set 203, in which case the database may be queried to detect when the intravenous set 203 is being used impermissibly when the intravenous set 203 has already being used and/or is past its expiration date.

In addition to misloads of the intravenous set 203, the machine learning engine 115 may be also be trained to identify other nonconformities such as the intravenous set 203 not being primed properly of air, a missing component in the pump 120 and/or the intravenous set 203, a damaged component in the pump 120 and/or the intravenous set 203, and the presence of contaminants in the pump 120 and/or the intravenous set 203. Examples of missing or damaged components in the intravenous set 203 may include the upper fitment 205, the pumping segment 207 of the tubing, and the lower fitment 209. Examples of missing or damaged components in the pump 120 may include the bezel, the membrane seal, one or more of the locator features204a and 204b, the door 206, the platen 208, the pumping mechanism 210, and the air-in-line detector 212.

As noted, the machine learning engine 115 may include one or more machine learning models that have been trained, based on images of correctly loaded intravenous sets, to detect a misload of the intravenous set 203. To do so, the machine learning engine 115 may first enhance the raw images captured by the camera 204 including by performing edge detection to determine the relative positions of the various components of the pump 120 and the intravenous set 203 depicted in the images captured by the camera 204. A Laplace-Gaussian transformation is one example edge detection technique that the machine learning engine 115 may apply to identify the edges that are present in the images of the pump 120 loaded with the intravenous set 203 and generate corresponding line segments. Canny edge detection is another edge detection technique that detects the edges present in the images of the pump 120 loaded with the intravenous set 203 by subjecting each image to noise reduction, gradient calculation (e.g., Gaussian blur), non-maximum suppression, double thresholding, and edge tracking by hysteresis. The enhanced images may be used for one or more downstream machine vision tasks such as feature extraction, object classification, and optical character recognition. For example, the machine learning engine 115 may compare an enhanced image of the pump 120 loaded with the intravenous set 203 with one or more reference images depicting correctly loaded intravenous sets to determine whether the images of the pump 120 exhibits one or more nonconformities. The various components identified within the images of the pump 120 loaded with the intravenous set 203 may serve as reference points for determining the spatial relationship between the various components. For instance, the machine learning engine 115 may identify the top fitment 205 of the intravenous set 203 as a first reference point and the first locator feature 202a as a second reference point in order to determine the spatial relationship between the top fitment 205 and the first locator feature 202a. In cases where at least some of the images captured by the camera 204 are stored, data privacy and storage efficiency may be maximized by the machine learning engine 115 storing a simplified representation of the images such as, for example, a vector of the coordinates of the various components present in the images.

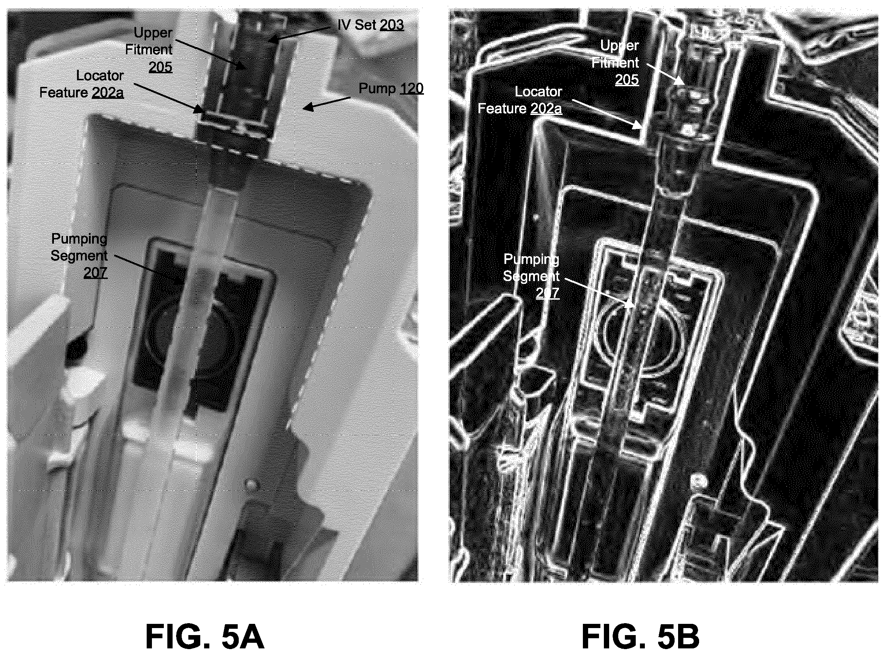

To further illustrate, FIG. 5A depicts a raw image illustrating a correctly loaded intravenous set 203 with the top fitment 205 of the intravenous set 203 positioned at the base of the first locator feature 202a (e.g., a pocket) in the bezel of the pump 120. FIG. 5B depicts an enhanced image that is generated by the machine learning engine 115 applying an edge detection technique to identify the position of various components of the pump 120 and the intravenous set 203 including the top fitment 205 and the first locator feature 202a.

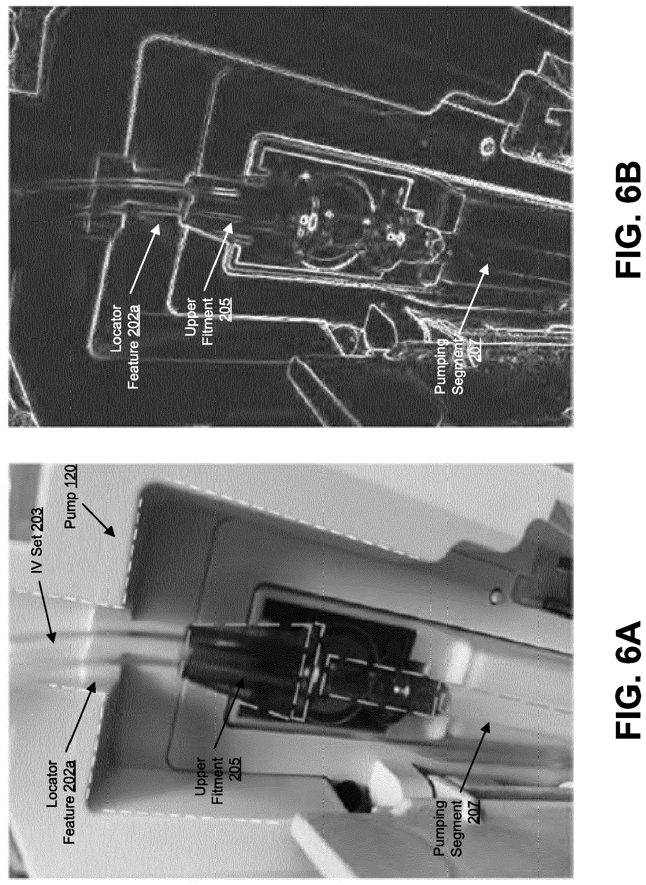

FIG. 6A depicts a raw image illustrating an incorrectly loaded intravenous set 203 with the top fitment 205 of the intravenous set 203 positioned below the first locator feature 202a (e.g., the pocket) in the bezel of the pump 120 and in an area of the pump 120 occupied by the upper pressure sensor. FIG. 6B depicts an enhanced image generated by the machine learning engine 115 applying an edge detection technique to identify the position of various components of the pump 120 and the intravenous set 203 including the top fitment 205 and the first locator feature 202a.

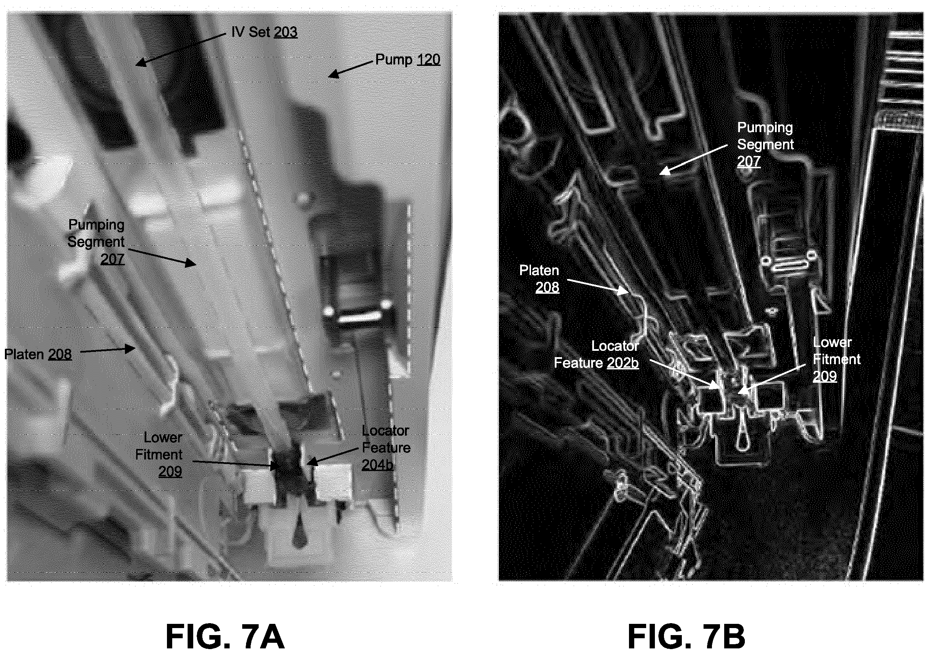

FIG. 7A depicts a raw image illustrating the intravenous set 203 loaded correctly in the pump 120 with the lower fitment 209 positioned in a second locator feature 202b and the flow stop in a closed position. The raw image depicted in FIG. 7A further shows the platen 208 (and various features thereon) such that the position of the platen 208 may be analyzed relative to that of the pump 120 (and various components therein) to ensure that the platen 208 will engage properly with the pump 120 and the loaded intravenous set 203 when the door 206 of the pump 120 is in a closed position. FIG. 7B depicts an enhanced image generated by the machine learning engine 115 applying an edge detection technique to identify the position of various components of the pump 120 and the intravenous set 203 including the platen 208, the bottom fitment 209, and the second locator feature 202b.

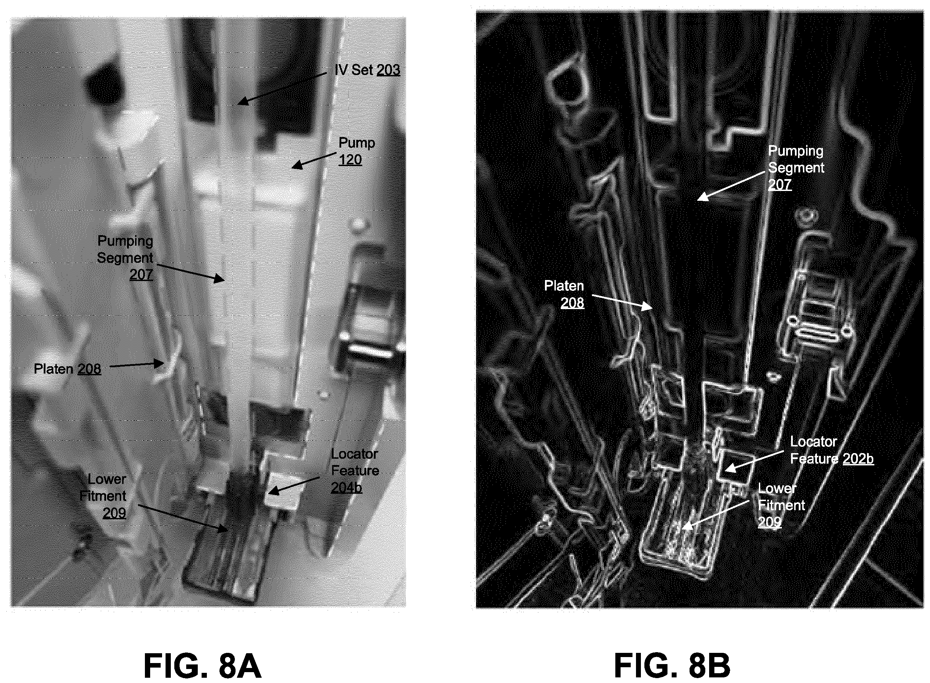

FIG. 8A depicts a raw image illustrating the intravenous set 203 loaded incorrectly in the pump 120 with the lower fitment 209 placed backwards in the cavity of the pump 120. An enhanced version of the raw image shown in FIG. 8A is depicted in FIG. 8B. The enhanced image shown in FIG. 8B may be generated by the machine learning engine 115 applying an edge detection technique to identify the position of various components of the pump 120 and the intravenous set 203 including the platen 208, the bottom fitment 209, and the second locator feature 202b.

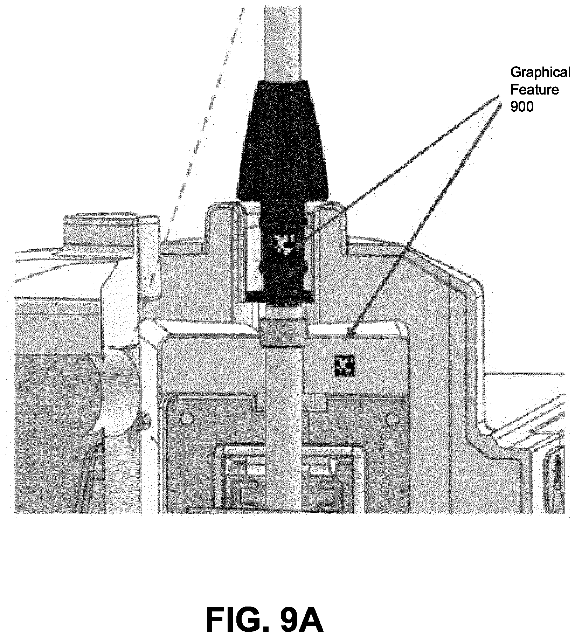

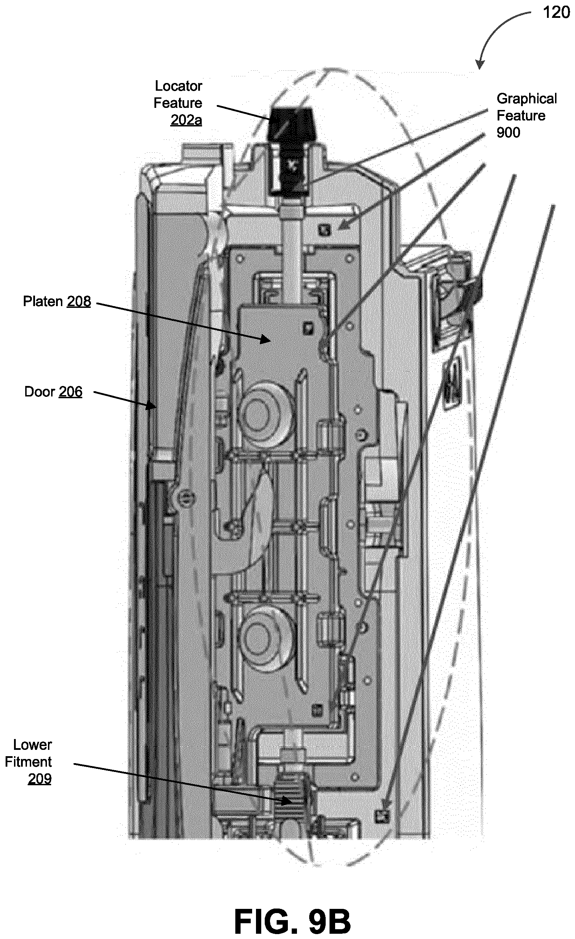

In some example embodiments, to identity various components of the pump 120 and the intravenous set 203, the machine learning engine 115 may be configured to recognize one or more graphical features such as barcodes, April Tags, and/or the like. FIGS. 9A-B depict the pump 120 with various examples of a graphical feature 900, which may be placed on different components of the pump 120 and the intravenous set 203 to provide reference points for determining the spatial relationships between, for example, the upper fitment 205, the platen 208, and/or the like. It should be appreciated that the graphical feature 900 may enable a 6 degree-of-freedom localization of the components that are present in the image. For example, the location and orientation of the graphical feature 900 on the upper fitment 205 relative to the location and orientation of the graphical feature 900 on the first locator feature 202a may indicate whether the upper fitment 205 is in a correct position relative to the first locator feature 202a. Where the graphical feature 900 is on one side (or surface) of a component such as the upper fitment 205, the quantity of the graphical feature 900 that is visible (or obscured) in the image may indicate whether, for example, the upper fitment 205 is placed in a correct orientation or is rotated in an incorrect position.

In some cases, the position of one or more the graphical features 900 in an image of the pump 120 loaded with the intravenous set 203 may also be used to correct for various forms of distortions that may be present in the image. For example, the image may exhibit a barrel distortion if the image is captured by the camera 204 when the camera 204 is too close to the intravenous set 203. The relative locations of the graphical features 900 in the image may be used to correct for the barrel distortion and other types of distortions that may be present in the image.

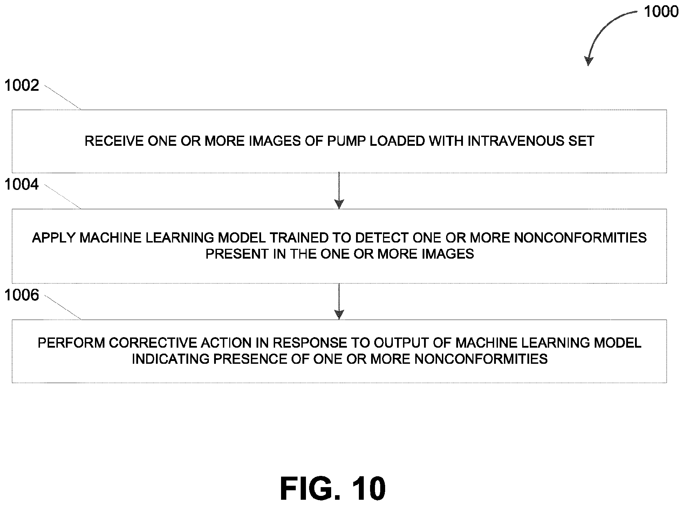

FIG. 10 depicts a flowchart illustrating an example of a process 1000 for machine learning enabled misload detection. Referring to FIGS. 1A and 10, the process 1000 may be performed by the pump controller 110 to detect and respond to a variety of anomalous conditions at the pump 120.

At 1002, the pump controller 110 may receive one or more images of a pump loaded with an intravenous set. In some example embodiments, the pump controller 110 may receive, from the camera 204, one or more images of the pump 120 loaded with the intravenous set 203. The camera 204 may be activated by the door 206 of the pump 120 being moved to an open position. The camera 204 may be further configured to capture images, for example, periodically, while the door 206 of the pump 120 remains in open position. As noted, the camera 204 may be configured to have a minimum field of view (FOV) such as, for example, 130° or the like. Moreover, the camera 204 may be mounted to in a location such that the field of view (FOV) of the camera 204 includes one or more areas of interest such as, for example, at least a portion of the platen 208 and the intravenous set 203 loaded in the pump 120 (e.g., the portion of the intravenous set 203 between an upper fitment 205 and a lower fitment 209), when the door 206 of the pump 120 is in an open position. In some cases, the field of view of the camera 204 may be limited to exclude certain areas including, for example, areas where surveillance is unsuitable, prohibited, or unnecessary.

At 1004, the pump controller 110 may apply a machine learning model trained to detect one or more nonconformities present in the one or more images. In some example embodiments, the machine learning engine 115 at the pump controller 110 may apply, to the images of the pump 120 loaded with the intravenous set 203, one or more machine learning models trained to perform a variety of machine vision tasks including, for example, image enhancement, morphological filtering, blob detection, feature extraction, image segmentation, edge detection, object classification, optical character recognition (OCR), and/or the like. For example, the machine learning engine 115 may enhance the raw images captured by the camera 204 by performing edge detection (e.g., Laplace-Gaussian transformation, Canny edge detection, and/or the like) to determine the relative positions of the various components of the pump 120 and the intravenous set 203 depicted therein. The machine learning engine 115 may compare the enhanced images of the pump 120 loaded with the intravenous set 203 with one or more reference images depicting correctly loaded intravenous sets to determine whether the images of the pump 120 exhibits one or more nonconformities.

The machine learning engine 115 may be configured to detect a variety of nonconformities present in the images of the pump 120 loaded with the intravenous set 203. One example of a nonconformity is a misload of the intravenous set 203 in which one or more portions of the intravenous set 203, such as the upper fitment 205, the pumping segment 207, and the lower fitment 209, are placed incorrectly in the pump 120. Another example of a nonconformity may be the intravenous set 203 being of an incorrect type, a counterfeit product, or an expired set. Other nonconformities may include the intravenous set 203 not being primed properly of air, a missing component in the pump 120 and/or the intravenous set 203, a damaged component in the pump 120 and/or the intravenous set 203, and the presence of contaminants in the pump 120 and/or the intravenous set 203.

At 1006, the pump controller 110 may perform a corrective action in response to an output of the machine learning model indicating a presence of one or more nonconformities in the one or more images of the pump loaded with the intravenous set. In some example embodiments, the pump controller 110 may perform one or more corrective actions when the output of the machine learning engine 115 indicates the presence of a nonconformity in the images of the pump 120 loaded with the intravenous set 203. For example, the pump controller 110 may prevent the pump 120 from performing an infusion while a nonconformity is determined to be present at the pump 120. Alternatively and/or additionally, the pump controller 110 may generate a message indicating, for example, a misload of the intravenous set 203 at the pump 120. In some cases, the message may provide instructions for correcting the misload including by identifying the type and location of the misload at the pump 120. The message may be presented via a user interface associated with the pump 120.

Performing a corrective action a device may include transmitting one or more messages to adjust an operational state or functional element of the device. The message may include specific instructions to be executed by a processor of the device to manifest the change. The corrective action may include storing a value in a location of a storage device for subsequent retrieval by the device to be controlled, transmitting a value directly to the device to be controlled via at least one wired or wireless communication medium, transmitting or storing a reference to a value, and the like. For example, a control message may include a value to adjust a level of power from a power source of the controlled device. As another example, a control message may activate or deactivate a structural element of the controlled device such as a light, audio playback, a motor, a lock, a pump, a power supply, a display, or other component of a device described herein. A corrective action may include indirect control of the device by adjusting a configuration value used by the controlled device. For example, the control message may include a threshold value for a device characteristic (e.g., temperature, rate, frequency, etc.). The threshold value may be stored in a memory location and referred to by the controlled device during operation

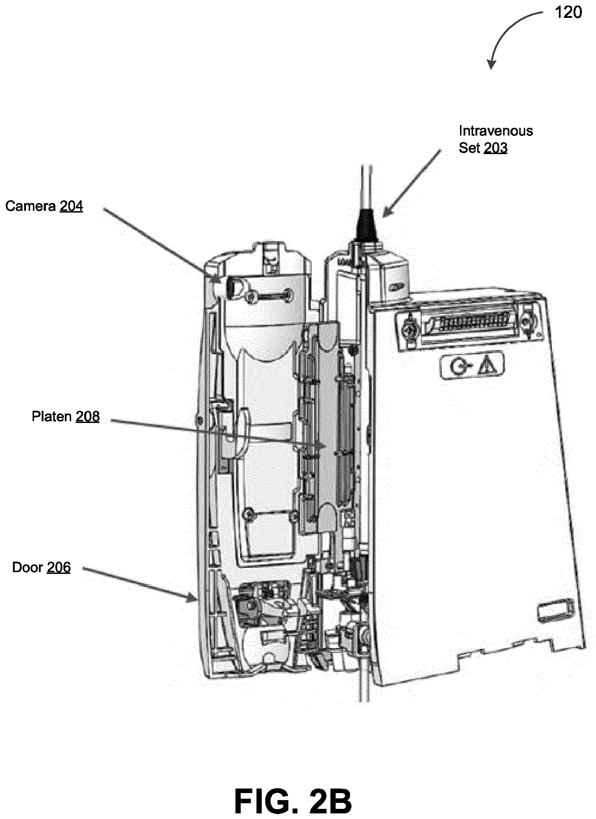

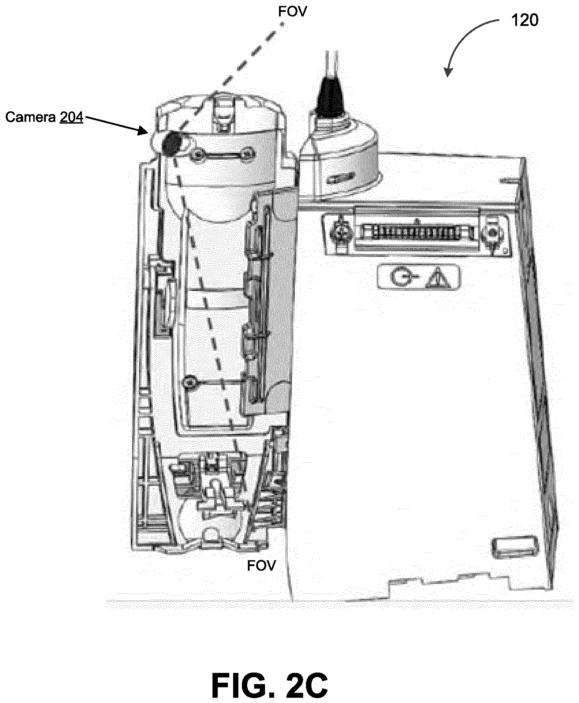

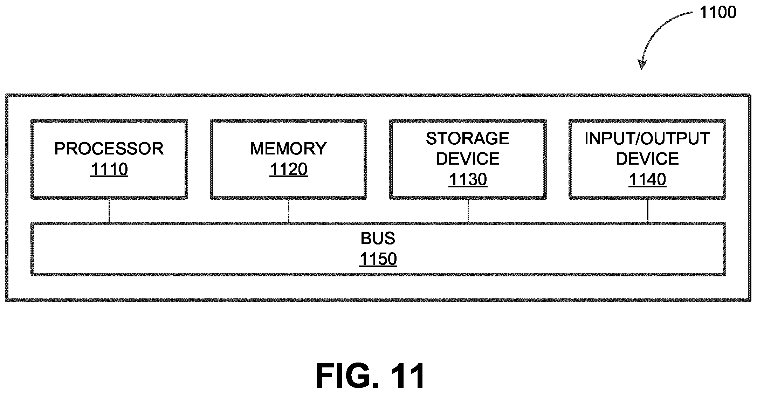

FIG. 11 depicts a block diagram illustrating a computing system 1100 consistent with implementations of the current subject matter. Referring to FIGS. 1 and 2, the computing system 1100 can be used to implement the pump controller 110 and/or any components therein.

As shown in FIG. 11, the computing system 1100 can include a processor 1110, a memory 1120, a storage device 1130, and input/output device 11401140. The processor 1110, the memory 1120, the storage device 1130, and the input/output device 1140 can be interconnected via a system bus 1150. The processor 1110 is capable of processing instructions for execution within the computing system 1100. Such executed instructions can implement one or more components of, for example, the pump controller 110. In some example embodiments, the processor 1110 can be a single-threaded processor. Alternatively, the processor 1110 can be a multi-threaded processor. The processor 1110 is capable of processing instructions stored in the memory 1120 and/or on the storage device 1130 to display graphical information for a user interface provided via the input/output device 1140.

The memory 1120 is a computer readable medium such as volatile or nonvolatile that stores information within the computing system 1100. The memory 1120 can store data structures representing configuration object databases, for example. The storage device 1130 is capable of providing persistent storage for the computing system 1100. The storage device 1130 can be a floppy disk device, a hard disk device, an optical disk device, a tape device, a solid-state device, and/or any other suitable persistent storage means. The input/output device 1140 provides input/output operations for the computing system 1100. In some example embodiments, the input/output device 1140 includes a keyboard and/or pointing device. In various implementations, the input/output device 1140 includes a display unit for displaying graphical user interfaces.

According to some example embodiments, the input/output device 1140 can provide input/output operations for a network device. For example, the input/output device 1140 can include Ethernet ports or other networking ports to communicate with one or more wired and/or wireless networks (e.g., a local area network (LAN), a wide area network (WAN), the Internet).

In some example embodiments, the computing system 1100 can be used to execute various interactive computer software applications that can be used for organization, analysis and/or storage of data in various formats. Alternatively, the computing system 1100 can be used to execute any type of software applications. These applications can be used to perform various functionalities, e.g., planning functionalities (e.g., generating, managing, editing of spreadsheet documents, word processing documents, and/or any other objects, etc.), computing functionalities, communications functionalities, etc. The applications can include various add-in functionalities or can be standalone computing products and/or functionalities. Upon activation within the applications, the functionalities can be used to generate the user interface provided via the input/output device 1140. The user interface can be generated and presented to a user by the computing system 1100 (e.g., on a computer screen monitor, etc.).

One or more aspects or features of the subject matter described herein can be realized in digital electronic circuitry, integrated circuitry, specially designed ASICs, field programmable gate arrays (FPGAs) computer hardware, firmware, software, and/or combinations thereof. These various aspects or features can include implementation in one or more computer programs that are executable and/or interpretable on a programmable system including at least one programmable processor, which can be special or general purpose, coupled to receive data and instructions from, and to transmit data and instructions to, a storage system, at least one input device, and at least one output device. The programmable system or computing system may include clients and servers. A client and server are generally remote from each other and typically interact through a communication network. The relationship of client and server arises by virtue of computer programs running on the respective computers and having a client-server relationship to each other.

These computer programs, which can also be referred to as programs, software, software applications, applications, components, or code, include machine instructions for a programmable processor, and can be implemented in a high-level procedural and/or object-oriented programming language, and/or in assembly/machine language. As used herein, the term "machine-readable medium" refers to any computer program product, apparatus and/or device, such as for example magnetic discs, optical disks, memory, and Programmable Logic Devices (PLDs), used to provide machine instructions and/or data to a programmable processor, including a machine-readable medium that receives machine instructions as a machine-readable signal. The term "machine-readable signal" refers to any signal used to provide machine instructions and/or data to a programmable processor. The machine-readable medium can store such machine instructions non-transitorily, such as for example as would a non-transient solid-state memory or a magnetic hard drive or any equivalent storage medium. The machine-readable medium can alternatively or additionally store such machine instructions in a transient manner, such as for example, as would a processor cache or other random access memory associated with one or more physical processor cores.

To provide for interaction with a user, one or more aspects or features of the subject matter described herein can be implemented on a computer having a display device, such as for example a cathode ray tube (CRT) or a liquid crystal display (LCD) or a light emitting diode (LED) monitor for displaying information to the user and a keyboard and a pointing device, such as for example a mouse or a trackball, by which the user may provide input to the computer. Other kinds of devices can be used to provide for interaction with a user as well. For example, feedback provided to the user can be any form of sensory feedback, such as for example visual feedback, auditory feedback, or tactile feedback; and input from the user may be received in any form, including acoustic, speech, or tactile input. Other possible input devices include touch screens or other touch-sensitive devices such as single or multi-point resistive or capacitive track pads, voice recognition hardware and software, optical scanners, optical pointers, digital image capture devices and associated interpretation software, and the like.

In the descriptions above and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." Use of the term "based on," above and in the claims is intended to mean, "based at least in part on," such that an unrecited feature or element is also permissible.

As used herein a "user interface" (also referred to as an interactive user interface, a graphical user interface or a UI) may refer to a network based interface including data fields and/or other control elements for receiving input signals or providing electronic information and/or for providing information to the user in response to any received input signals. Control elements may include dials, buttons, icons, selectable areas, or other perceivable indicia presented via the UI that, when interacted with (e.g., clicked, touched, selected, etc.), initiates an exchange of data for the device presenting the UI. A UI may be implemented in whole or in part using technologies such as hyper-text mark-up language (HTML), FLASH^{™}, JAVA^{™}, .NET^{™}, C, C++, web services, or rich site summary (RSS). In some embodiments, a UI may be included in a stand-alone client (for example, thick client, fat client) configured to communicate (e.g., send or receive data) in accordance with one or more of the aspects described. The communication may be to or from a medical device or server in communication therewith.

The subject matter described herein can be embodied in systems, apparatus, methods, and/or articles depending on the desired configuration. The implementations set forth in the foregoing description do not represent all implementations consistent with the subject matter described herein. Instead, they are merely some examples consistent with aspects related to the described subject matter. Although a few variations have been described in detail above, other modifications or additions are possible. In particular, further features and/or variations can be provided in addition to those set forth herein. For example, the implementations described above can be directed to various combinations and subcombinations of the disclosed features and/or combinations and subcombinations of several further features disclosed above. In addition, the logic flows depicted in the accompanying figures and/or described herein do not necessarily require the particular order shown, or sequential order, to achieve desirable results. Other implementations may be within the scope of the following claims.

## Claims

1. A system (1100), comprising:
at least one data processor (1110); and
at least one memory (1120) storing instructions which, when executed by the at least one data processor (1110), result in operations comprising:
receiving, from a camera (204) at an infusion pump (120), one or more images of an area of interest of the infusion pump (120) loaded with an infusion set (203);
identifying, within the one or more images, a first component of the infusion pump (120) and a second component of the infusion set (203) in the area of interest, wherein the first component comprises a bezel, a membrane seal, a door (206), a platen (208), a locator feature (202a, 202b), or an air-in-line detector (212), and wherein the second component comprises an upper fitment (205), a lower fitment (209), or a pumping segment (207) of a tubing;
applying a machine learning model trained to detect one or more nonconformities present in the one or more images of the infusion pump (120) loaded with the infusion set (203) based at least on a relative position of the first component and the second component and by at least comparing, to one or more images of a correctly loaded infusion set (203), the one or more images of the infusion pump (120) loaded with the infusion set (203); and
in response to an output of the machine learning model indicating a presence of a nonconformity in the one or more images of the infusion pump (120) loaded with the infusion set (203), performing a corrective action.

2. The system (1100) of claim 1, wherein the first component and the second component are identified by (i) applying an edge detection technique and/or (ii) based on a graphical feature (900) disposed on each one of the first component and the second component.

3. The system (1100) of claim 1 or 2, wherein the one or more nonconformities include at least one of (i) a misload of the infusion set (203) including a misplacement of an upper fitment (205) of the infusion set (203), a lower fitment (209) of the infusion set (203), and/or a pumping segment (207) of a tubing of the infusion set (203), (ii) the intravenous set (203) being reused, past an expiration date, an incorrect type, or a counterfeit product, (iii) one or more components of the infusion pump (120) and/or the intravenous set (203) being missing and/or damaged, and (iv) a presence of contaminants in the infusion pump (120) and/or the intravenous set (203).

4. The system (1100) of any of claims 1 to 3, wherein the camera (204) is mounted to a door (206) of the infusion pump (120), wherein the camera (204) is mounted in a location where the camera (204) has a field of view that includes at least a portion of the infusion pump (120) loaded with the intravenous set (203), and wherein the field of view of the camera (204) optionally excludes one or more areas surveillance is unsuitable, prohibited, or unnecessary.

5. The system (1100) of claim 4, wherein the one or more images include a first image captured while the door (206) is in an open position and a second image captured while the door (206) is in a partially open position, and wherein the one or more nonconformities are detected based on the first image and the second image.

6. The system of any of claims 1 to 5, wherein the camera (204) comprises a visible light camera, an infrared camera, and/or an ultraviolet camera, and wherein the one or more nonconformities are detected based on a graphical feature (900) that is detectable under visible light, infrared light, or ultraviolet light.

7. The system of any of claims 1 to 6, wherein the corrective action includes at least one of (i) preventing the infusion pump (120) from performing an infusion and (ii) generating a message identifying the one or more nonconformities.

8. An infusion pump (120), comprising:
a bezel having one or more locator features (202a, 202b) for receiving an intravenous set (203);
a camera (204) mounted to a door (206) of the infusion pump (120), the camera (120) being mounted in a location where the camera (204) has a field of view that includes an area of interest with at least a portion of the infusion pump (120) loaded with the intravenous set (203) and where the field of view optionally excludes one or more areas surveillance is unsuitable, prohibited, or unnecessary, and the camera (204) being configured to capture one or more images of the infusion pump (120) loaded with the intravenous set (203); and
a controller (110) comprising at least data processor (1110) and at least one memory (1120) storing instructions which, when executed by the at least one data processor (1110), cause the controller (110) to perform operations comprising:
identifying, within the one or more images, a first component of the infusion pump (120) and a second component of the infusion set (203) within the area of interest, the first component comprising the bezel, the door (206), the one or more locator features (202a, 202b), a membrane seal, a platen (208), or an air-in-line detector (212), and wherein the second component comprises an upper fitment (205), a lower fitment (209), or a pumping segment (207) of a tubing;
applying a machine learning model trained to detect one or more nonconformities present in the one or more images of the infusion pump (120) loaded with the infusion set (203) based at least on a relative position of the first component and the second component and by at least comparing, to one or more images of a correctly loaded infusion set (203), the one or more images of the infusion pump (120) loaded with the infusion set (203); and
in response to an output of the machine learning model indicating a presence of a nonconformity in the one or more images of the infusion pump (120) loaded with the infusion set (203), performing a corrective action.

9. The infusion pump (120) of claim 8, wherein the first component and the second component are identified by (i) applying an edge detection technique and/or (ii) based on a graphical feature (900) disposed on each one of the first component and the second component.

10. The infusion pump (120) of claim 8 or 9, wherein the one or more nonconformities include at least one of (i) a misload of the infusion set (203) including a misplacement of an upper fitment (205) of the infusion set (203), a lower fitment (209) of the infusion set (203), and/or a pumping segment (207) of a tubing of the infusion set (203), (ii) the intravenous set (203) being reused, past an expiration date, an incorrect type, or a counterfeit product, (iii) one or more components of the infusion pump (120) and/or the intravenous set (203) being missing and/or damaged, and (iv) a presence of contaminants in the infusion pump (120) and/or the intravenous set (203).

## Patentansprüche

1. Ein System (1100), umfassend:
mindestens einen Datenprozessor (1110); und
mindestens einen Speicher (1120), der Befehle speichert, die, wenn sie von dem mindestens einen Datenprozessor (1110) ausgeführt werden, zu Operationen führen, die umfassen:
Empfangen von einer Kamera (204) an einer Infusionspumpe (120) von einem oder mehreren Bildern eines Bereichs von Interesse der Infusionspumpe (120), die mit einem Infusionsset (203) beladen ist;
Identifizieren einer ersten Komponente der Infusionspumpe (120) und einer zweiten Komponente des Infusionssets (203) in dem Bereich von Interesse innerhalb des einen oder mehreren Bildern, wobei die erste Komponente eine Blende, eine Membrandichtung, eine Tür (206), eine Platte (208), ein Lokalisierungsmerkmal (202a, 202b) oder einen Luft-in-Leitung-Detektor (212) umfasst, und wobei die zweite Komponente ein oberes Anschlussstück (205), ein unteres Anschlussstück (209) oder ein Pumpsegment (207) eines Schlauchs umfasst;
Anwenden eines maschinellen Lernmodells, das trainiert wurde, um eine oder mehrere Nichtkonformitäten zu erkennen, die in dem einen oder mehreren Bildern der mit dem Infusionsset (203) beladenen Infusionspumpe (120) vorhanden sind, basierend auf mindestens einer relativen Position der ersten Komponente und der zweiten Komponente und mindestens durch Vergleichen des einen oder mehreren Bildern der mit dem Infusionsset (203) beladenen Infusionspumpe (120) mit einem oder mehreren Bildern eines korrekt beladenen Infusionssets (203); und
in Antwort auf eine Ausgabe des maschinellen Lernmodells, die ein Vorhandensein einer Nichtkonformität in dem einen oder mehreren Bildern der mit dem Infusionsset (203) beladenen Infusionspumpe (120) anzeigt, Durchführen einer Korrekturmaßnahme.

2. System (1100) nach Anspruch 1, wobei die erste Komponente und die zweite Komponente identifiziert werden, indem (i) eine Kantenerkennungstechnik angewandt wird und/oder (ii) basierend auf einem grafischen Merkmal (900), das auf jeder der ersten Komponente und der zweiten Komponente angeordnet ist.

3. System (1100) nach Anspruch 1 oder 2, wobei die eine oder mehreren Nichtkonformitäten mindestens eines von enthalten: (i) eine Fehlbeladung des Infusionssets (203) einschließlich einer Fehlplatzierung eines oberen Anschlusses (205) des Infusionssets (203), eines unteren Anschlusses (209) des Infusionssets (203) und/oder eines Pumpsegments (207) eines Schlauchs des Infusionssets (203), (ii) das intravenöse Set (203), das wiederverwendet wird, das ein Verfallsdatum überschritten hat, das ein falscher Typ oder ein gefälschtes Produkt ist, (iii) eine oder mehrere Komponenten der Infusionspumpe (120) und/oder des intravenösen Sets (203), die fehlen und/oder beschädigt sind, und (iv) ein Vorhandensein von Verunreinigungen in der Infusionspumpe (120) und/oder dem intravenösen Set (203).

4. System (1100) nach einem der Ansprüche 1 bis 3, wobei die Kamera (204) an einer Tür (206) der Infusionspumpe (120) angebracht ist, wobei die Kamera (204) an einem Ort angebracht ist, wo die Kamera (204) ein Sichtfeld hat, das mindestens einen Teil der mit dem intravenösen Set (203) beladenen Infusionspumpe (120) enthält, und wobei das Sichtfeld der Kamera (204) optional einen oder mehrere Bereiche ausschließt, wo Überwachung ungeeignet, verboten oder unnötig ist.

5. System (1100) nach Anspruch 4, wobei das eine oder mehrere Bilder ein erstes Bild enthalten, das aufgenommen wurde, während sich die Tür (206) in einer offenen Position befindet, und ein zweites Bild, das aufgenommen wurde, während sich die Tür (206) in einer teilweise offenen Position befindet, und wobei die eine oder mehrere Nichtkonformitäten basierend auf dem ersten Bild und dem zweiten Bild erkannt werden.

6. System nach einem der Ansprüche 1 bis 5, wobei die Kamera (204) eine Kamera für sichtbares Licht, eine Infrarotkamera und/oder eine Ultraviolettkamera umfasst, und wobei die eine oder mehreren Nichtkonformitäten basierend auf einem grafischen Merkmal (900) erkannt werden, das unter sichtbarem Licht, Infrarotlicht oder Ultraviolettlicht erkennbar ist.

7. System nach einem der Ansprüche 1 bis 6, wobei die Korrekturmaßnahme mindestens eines von enthält: (i) Verhindern, dass die Infusionspumpe (120) eine Infusion durchführt, und (ii) Erzeugen einer Meldung, die die eine oder mehrere Nichtkonformitäten identifiziert.

8. Eine Infusionspumpe (120) umfassend:
eine Blende aufweisend ein oder mehrere Lokalisierungsmerkmale (202a, 202b) zur Aufnahme eines intravenösen Sets (203);
eine Kamera (204), die an einer Tür (206) der Infusionspumpe (120) angebracht ist, wobei die Kamera (120) an einer Stelle angebracht ist, wo die Kamera (204) ein Sichtfeld hat, das einen Bereich von Interesse mit mindestens einem Teil der mit dem intravenösen Set (203) beladenen Infusionspumpe (120) enthält, und wo das Sichtfeld optional einen oder mehrere Bereiche ausschließt, wo Überwachung ungeeignet, verboten oder unnötig ist, und wobei die Kamera (204) dazu konfiguriert ist, ein oder mehrere Bilder der mit dem intravenösen Set (203) beladenen Infusionspumpe (120) aufzunehmen; und
eine Steuerung (110), die mindestens einen Datenprozessor (1110) und mindestens einen Speicher (1120) umfasst, der Anweisungen speichert, die, wenn sie von dem mindestens einen Datenprozessor (1110) ausgeführt werden, die Steuerung (110) dazu veranlassen, Operationen durchzuführen, die umfassen:
Identifizieren einer ersten Komponente der Infusionspumpe (120) und einer zweiten Komponente des Infusionssets (203) im des Bereichs von Interesse in dem einen oder mehreren Bildern, wobei die erste Komponente die Blende, die Tür (206), das eine oder mehrere Lokalisierungsmerkmale (202a, 202b), eine Membrandichtung, eine Platte (208) oder einen Luft-in-Leitung-Detektor (212) umfasst, und wobei die zweite Komponente ein oberes Anschlussstück (205), ein unteres Anschlussstück (209) oder ein Pumpsegment (207) eines Schlauchs umfasst;
Anwenden eines maschinellen Lernmodells, das trainiert wurde, um eine oder mehrere Nichtkonformitäten zu erkennen, die in dem einen oder mehreren Bildern der mit dem Infusionsset (203) beladenen Infusionspumpe (120) vorhanden sind, basierend auf mindestens einer relativen Position der ersten Komponente und der zweiten Komponente und mindestens durch Vergleichen des einen oder mehreren Bildern der mit dem Infusionsset (203) beladenen Infusionspumpe (120) mit einem oder mehreren Bildern eines korrekt beladenen Infusionssets (203); und
in Reaktion auf eine Ausgabe des maschinellen Lernmodells, die ein Vorhandensein einer Nichtkonformität in dem einen oder mehreren Bildern der mit dem Infusionsset (203) beladenen Infusionspumpe (120) anzeigt, Durchführen einer Korrekturmaßnahme.

9. Infusionspumpe (120) nach Anspruch 8, wobei die erste Komponente und die zweite Komponente identifiziert werden, indem (i) eine Kantenerkennungstechnik angewendet wird und/oder (ii) basierend auf einem grafischen Merkmal (900), das auf jeder der ersten Komponente und der zweiten Komponente angeordnet ist.

10. Infusionspumpe (120) nach Anspruch 8 oder 9, wobei die eine oder mehreren Nichtkonformitäten mindestens eines von enthalten: (i) eine Fehlbeladung des Infusionssets (203) einschließlich einer Fehlplatzierung eines oberen Anschlusses (205) des Infusionssets (203), eines unteren Anschlusses (209) des Infusionssets (203) und/oder eines Pumpsegments (207) eines Schlauchs des Infusionssets (203), (ii) das intravenöse Set (203), das wiederverwendet wird, das ein Verfallsdatum überschritten hat, das ein falscher Typ oder ein gefälschtes Produkt ist, (iii) eine oder mehrere Komponenten der Infusionspumpe (120) und/oder des intravenösen Sets (203), die fehlen und/oder beschädigt sind, und (iv) ein Vorhandensein von Verunreinigungen in der Infusionspumpe (120) und/oder dem intravenösen Set (203).

## Revendications

1. Un Système (1100) comprenant
au moins un processeur de données (1110) ; et
au moins une mémoire (1120) stockant des instructions qui, lorsqu'elles sont exécutées par au moins un processeur de données (1110), entraînent des opérations comprenant :
recevoir, d'une caméra (204) située sur une pompe à perfusion (120), une ou plusieurs images d'une zone d'intérêt de la pompe à perfusion (120) chargée d'un ensemble de perfusion (203) ;
identifier, dans l'une ou plusieurs images, un premier composant de la pompe à perfusion (120) et un second composant de l'ensemble de perfusion (203) dans la zone d'intérêt, le premier composant comprenant une lunette, un joint de membrane, une porte (206), un plateau (208), un élément de localisation (202a, 202b) ou un détecteur d'entrée d'air (212), et le second composant comprenant un raccord supérieur (205), un raccord inférieur (209) ou un segment de pompage (207) d'une tubulure ;
appliquer un modèle d'apprentissage automatique formé pour détecter une ou plusieurs non-conformités présentes dans l'une ou plusieurs images de la pompe à perfusion (120) chargée de l'ensemble de perfusion (203) en se basant au moins sur une position relative du premier composant et du second composant et au moins en comparant, à une ou plusieurs images d'un ensemble de perfusion correctement chargé (203), l'une ou plusieurs images de la pompe à perfusion (120) chargée de l'ensemble de perfusion (203) ; et
en réponse à une sortie du modèle d'apprentissage automatique indiquant la présence d'une non-conformité dans l'une ou plusieurs images de la pompe à perfusion (120) chargée de l'ensemble de perfusion (203), effectuer une action corrective.

2. Le système (1100) de la revendication 1, dans lequel le premier composant et le second composant sont identifiés (i) en appliquant une technique de détection des bords et/ou (ii) sur la base d'un élément graphique (900) disposée sur chacun du premier composant et du second composant.

3. Le système (1100) de la revendication 1 ou 2, dans lequel l'une ou plusieurs non-conformités comprennent au moins l'un des éléments suivants (i) une mauvaise charge de l'ensemble de perfusion (203), y compris un mauvais positionnement d'un raccord supérieur (205) de l'ensemble de perfusion (203), d'un raccord inférieur (209) de l'ensemble de perfusion (203), et/ou d'un segment de pompage (207) d'une tubulure de l'ensemble de perfusion (203), (ii) l'ensemble intraveineux (203) est réutilisé, sa date de péremption est dépassée, il est d'un type incorrect ou il est un produit contrefait, (iii) un ou plusieurs composants de la pompe à perfusion (120) et/ou de l'ensemble intraveineux (203) sont manquants et/ou endommagés, et (iv) une présence de contaminants dans la pompe à perfusion (120) et/ou dans l'ensemble intraveineux (203).

4. Le système (1100) de l'une des revendications 1 à 3, dans lequel la caméra (204) est montée sur une porte (206) de la pompe à perfusion (120), dans lequel la caméra (204) est montée à un endroit où la caméra (204) a un champ de vision qui inclut au moins une partie de la pompe à perfusion (120) chargée de l'ensemble intraveineux (203), et dans lequel le champ de vision de la caméra (204) exclut optionnellement une ou plusieurs zones où la surveillance est inappropriée, interdite ou inutile.

5. Le système (1100) de la revendication 4, dans lequel l'une ou plusieurs images comprennent une première image capturée lorsque la porte (206) est en position ouverte et une seconde image capturée lorsque la porte (206) est en position partiellement ouverte, et dans lequel l'une ou plusieurs non-conformités sont détectées sur la base de la première image et de la seconde image.

6. Le système de l'une des revendications 1 à 5, dans lequel la caméra (204) comprend une caméra à lumière visible, une caméra infrarouge et/ou une caméra ultraviolette, et dans lequel l'une ou plusieurs non-conformités sont détectées sur la base d'une caractéristique graphique (900) détectable à la lumière visible, à la lumière infrarouge ou à la lumière ultraviolette.

7. Le système de l'une des revendications 1 à 6, dans lequel l'action corrective comprend au moins l'une des actions suivantes : (i) empêcher la pompe à perfusion (120) d'effectuer une perfusion et (ii) générer un message identifiant l'une ou plusieurs non-conformités.

8. Pompe à perfusion (120), comprenant :
une lunette comportant un ou plusieurs éléments de localisation (202a, 202b) pour recevoir un ensemble intraveineux (203) ;
une caméra (204) montée sur une porte (206) de la pompe à perfusion (120), la caméra (120) étant montée à un endroit où la caméra (204) a un champ de vision qui inclut une zone d'intérêt avec au moins une partie de la pompe à perfusion (120) chargée de l'ensemble intraveineux (203) et où le champ de vision exclut éventuellement une ou plusieurs zones où la surveillance est inappropriée, interdite ou inutile, et la caméra (204) étant configurée pour capturer une ou plusieurs images de la pompe à perfusion (120) chargée de l'ensemble intraveineux (203) ; et
un contrôleur (110) comprenant au moins un processeur de données (1110) et au moins une mémoire (1120) stockant des instructions qui, lorsqu'elles sont exécutées par l'au moins un processeur de données (1110), amènent le contrôleur (110) à effectuer des opérations comprenant
identifier, dans l'une ou plusieurs images, un premier composant de la pompe à perfusion (120) et un second composant de l'ensemble de perfusion (203) dans la zone d'intérêt, le premier composant comprenant la lunette, la porte (206), un ou plusieurs éléments de localisation (202a, 202b), un joint de membrane, un plateau (208) ou un détecteur d'entrée d'air (212), et dans lequel le second composant comprend un raccord supérieur (205), un raccord inférieur (209) ou un segment de pompage (207) d'une tubulure
appliquer un modèle d'apprentissage automatique formé pour détecter une ou plusieurs non-conformités présentes dans l'une ou plusieurs images de la pompe à perfusion (120) chargée de l'ensemble de perfusion (203) en se basant au moins sur une position relative du premier composant et du second composant et au moins en comparant, à une ou plusieurs images de l'ensemble de perfusion correctement chargé (203), l'une ou plusieurs images de la pompe à perfusion (120) chargée de l'ensemble de perfusion (203) ; et
en réponse à une sortie du modèle d'apprentissage automatique indiquant la présence d'une non-conformité dans l'une ou plusieurs images de la pompe à perfusion (120) chargée de l'ensemble de perfusion (203), effectuer une action corrective.

9. La pompe à perfusion (120) de la revendication 8, dans laquelle le premier composant et le second composant sont identifiés (i) en appliquant une technique de détection des bords et/ou (ii) sur la base d'un élément graphique (900) disposée sur chacun du premier composant et du second composant.

10. La pompe à perfusion (120) de la revendication 8 ou 9, dans laquelle l'une ou plusieurs non-conformités comprennent au moins l'un des éléments suivants (i) une mauvaise charge de l'ensemble de perfusion (203), y compris un mauvais positionnement d'un raccord supérieur (205) de l'ensemble de perfusion (203), d'un raccord inférieur (209) de l'ensemble de perfusion (203), et/ou d'un segment de pompage (207) d'une tubulure de l'ensemble de perfusion (203), (ii) l'ensemble intraveineux (203) est réutilisé, sa date de péremption est dépassée, il est d'un type incorrect ou il est un produit contrefait, (iii) un ou plusieurs composants de la pompe à perfusion (120) et/ou de l'ensemble intraveineux (203) sont manquants et/ou endommagés, et (iv) une présence de contaminants dans la pompe à perfusion (120) et/ou dans l'ensemble intraveineux (203).
